(19) Europäisches Patentamt
European Patent Office
Office européen des brevets

(11) **EP 4 441 508 B1**

(12) **EUROPEAN PATENT SPECIFICATION**

(45) Date of publication and mention
of the grant of the patent:
**15.04.2026 Bulletin 2026/16**

(21) Application number: **22830191.7**

(22) Date of filing: **02.12.2022**

(51) International Patent Classification (IPC):
*G01N 33/68* (2006.01)    *A61B 5/055* (2006.01)

(52) Cooperative Patent Classification (CPC):
**G01N 33/6896; A61B 5/055; A61B 5/369;**
**A61B 5/4076; G01N 33/6866;** G01N 2333/7156;
G01N 2800/285; G01N 2800/52

(86) International application number:
**PCT/EP2022/084300**

(87) International publication number:
**WO 2023/099773 (08.06.2023 Gazette 2023/23)**

(54) **IN VITRO METHOD FOR THE DIAGNOSIS AND/OR PROGNOSIS OF MULTIPLE SCLEROSIS**

IN-VITRO-VERFAHREN ZUR DIAGNOSE UND/ODER PROGNOSE VON MULTIPLER SKLEROSE

PROCÉDÉ IN VITRO DE DIAGNOSTIC ET/OU DE PRONOSTIC DE LA SCLÉROSE EN PLAQUES

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**MD**

(30) Priority: **02.12.2021 EP 21383087**

(43) Date of publication of application:
**09.10.2024 Bulletin 2024/41**

(73) Proprietors:
• **ALA Diagnostics, S.L.**
**28006 Madrid (ES)**
• **Servicio Andaluz de Salud**
**41071 Sevilla (ES)**
• **Universidad De Malaga**
**E-29071 Málaga (ES)**

(72) Inventors:
• **FERNÁNDEZ FERNÁNDEZ, Óscar**
**28006 Madrid (ES)**
• **MONTERO MARTÍN-CALOTO, José Ramón**
**28006 Madrid (ES)**
• **PAVÍA MOLINA, José**
**29010 Málaga (ES)**

• **BARÓN LÓPEZ, Francisco Javier**
**29010 Málaga (ES)**
• **OLIVER MARTOS, Begoña**
**29010 Málaga (ES)**
• **HURTADO GUERRERO, Isaac**
**29010 Málaga (ES)**
• **SERRANO CASTRO, Pedro Jesús**
**29010 Málaga (ES)**
• **ALONSO TORRES, Ana**
**29010 Málaga (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(56) References cited:
**US-A1- 2016 011 212**

• **MARTINELLI V ET AL: "Multiple biomarkers**
**improve the prediction of multiple sclerosis in**
**clinically isolated syndromes", ACTA**
**NEUROLOGICA SCANDINAVICA,**
**MUNKSGAARD, COPENHAGEN, DK, vol. 136, no.**
**5, 9 April 2017 (2017-04-09), pages 454 - 461,**
**XP071063087, ISSN: 0001-6314, DOI: 10.1111/**
**ANE.12761**

   **(Cont. next page)**

- ÓRPEZ-ZAFRA T ET AL: "Development and validation of an ELISA for quantification of soluble IFN-beta receptor: assessment in multiple sclerosis", BIOANALYSIS, FUTURE SCIENCE, LONDON, UK, vol. 7, no. 22, 16 November 2015 (2015-11-16), pages 2869 - 2880, XP002756854, ISSN: 1757-6180, DOI: 10.4155/ BIO.15.208

**Description**

**FIELD OF THE INVENTION**

The present invention refers to the medical field. Particularly, the present invention refers to an *in vitro* method for the diagnosis and/or prognosis of multiple sclerosis (MS) in a subject which comprises assessing the concentration level of sIFNAR2 in a biological sample obtained from the subject in combination with the obtention of a magnetic resonance imaging (MRI) to assess the presence/absence of dissemination in space (MRI_DS) and/or dissemination in time (MRI_DT).

**STATE OF THE ART**

MS is an inflammatory, demyelinating and neurodegenerative disease that affects individuals with a genetic predisposition who have been exposed to an unknown agent, most likely infectious, which triggers an autoimmune process directed against the myelin that surrounds axons in the central nervous system (CNS), causing multiple lesions in various locations. This results in a wide range of symptoms, involving a significant clinical variability that hinders diagnosis. MS affects 2.8 million patients worldwide, thus being the most frequent cause of non-traumatic disability in young adults. Incidence can vary, between 2-8/100,000 inhabitants, depending on the country.

To date, several clinical phenotypes have been identified, from the earliest clinical form, i.e., when the first clinical manifestation characteristic of the disease appears (optic neuritis, brainstem syndrome or incomplete medullary lesion), known as clinically isolated syndrome (CIS). If the patient exhibits another episode of clinical activity or magnetic resonance imaging (MRI) activity, be it as lesions that take up gadolinium (Gad+) in the T1-weighted MRI sequence or be it as new or enlarged lesions in the T2-weighted MRI sequence (active lesions), then the patient has entered the relapsing-remitting MS (RRMS) phase, characterized by recurrent episodes of clinical activity (flare-ups and relapses) or MRI activity, with or without complete recovery or with subsequent sequelae. After 10-15 years of progression, approximately 80% of patients progress to a phase where generally there is a slow progression of the disability, with or without clinical worsening or flare-ups. This phase is known as secondary progressive MS (SPMS). Approximately 90% of patients exhibit this progression pattern. A 10% of patients exhibit a progressive course of disability from the onset of disease, with or without overlapping flare-ups, a clinical form known as primary progressive MS (PPMS).

In addition to the above-mentioned clinical forms, some people that for various reasons have had an MRI scan show characteristic MS lesions within the CNS, primarily in the brain. Therefore, this is a preclinical form known as radiologically isolated syndrome (RIS). It has been observed that many of these people may have data suggesting a certain level of neurodegeneration, which may suggest that the disease is active even years before the first neurological clinical manifestation appears.

Finally, in recent years, it has been observed, through retrospective studies, that MS patients visit their healthcare provider more frequently than healthy controls, due to various conditions or complaints, not clearly related with the CNS, having the term "prodromal MS" been coined, which means that onset of MS would actually occur many years before the first clinical manifestations. This is of particular relevance, since in order to know the cause(s) of the disease, it may be necessary to examine events occurred during adolescence, presumably the age at which the pathogenic manifestations that led to or triggered MS first appeared.

Despite increased knowledge and awareness of physicians on MS, diagnosis remains a challenge, even for experienced neurologists. Currently, no test is 100% specific for a MS diagnosis, and identification of accurate biomarkers is a challenge due to the clinical and pathophysiological complexities of the disease

So, there is an unmet medical need of finding reliable strategies which can be used for the diagnosis of MS with the highest possible sensitivity and specificity.

The present invention is thus focused on solving this problem and, consequently, a reliable strategy is herein provided, able to diagnose MS with an improved sensitivity and specificity as compared with current standard techniques.

MARTINELLI VET AL: "Multiple biomarkers improve the prediction of multiple sclerosis in clinically isolated syndromes", ACTA NEUROLOGICA SCANDINAVICA, MUNKSGAARD, COPENHAGEN, DK, vol. 136, no. 5, 9 April 2017(2017-04-09), pages 454-461, XP071063087, ISSN: 0001-6314, DOI: 10.1111/ANE.12761 discloses that the combination of variables and biomarkers, including MRI (magnetic resonance imaging), cerebrospinal fluid (CSF) data and Evoked Potentials improved prognosis of multiple sclerosis (MS) over established criteria. Among the MRI variables dissemination in time was the best single predictor and the presence of IgG oligoclonal bands (OCBs) was the most important fluid biomarker. It also discloses the effect-size of combining multiple biomarkers.

US 2016/011212 discloses soluble IFN(interferon)-B receptor (sIFNAR2) for use in diagnosis of multiple sclerosis, including in a kit. The document also discloses the addition of another marker (e.g. oligoclonal IgG band) to sIFNAR2 to improve the discrimination capacity and MS diagnosis as described with other biomarkers. The document also discloses the biomarkers spatial and temporal dissemination to confirm transition of patients from clinically isolated syndrome (CIS) to clinically defined multiple sclerosis (CDMS) and the use of sFNAR2 as an earlier biomarker for said transition.

None of the cited prior art discloses the unexpected synergistic effect observed in the present invention when IFNAR2 was combined with at least with MRI_DS and/or MRI_DT.

## DESCRIPTION OF THE INVENTION

### Brief description of the invention

The present invention is focused on assessing new strategies for the diagnosis of MS based on the determination of sIFNAR2 levels [Órpez-Zafra T, Pavía J, Pinto-Medel MJ, Hurtado-Guerrero I, Rodriguez-Bada JL, Martin Montañez E, Fernández Ó, Leyva L, Oliver-Martos B. Development and validation of an ELISA for quantification of soluble IFN-β receptor: assessment in multiple sclerosis. Bioanalysis. 2015;7(22):2869-2880] and [Órpez-Zafra T, Pavía J, Hurtado-Guerrero I, Pinto-Medel MJ, Rodriguez Bada JL, Urbaneja P, Suardíaz M, Villar LM, Comabella M, Montalban X, Alvarez-Cermeño JC, Leyva L, Fernández Ó, Oliver-Martos B. Decreased soluble IFN-β receptor (sIFNAR2) in multiple sclerosis patients: A potential serum diagnostic biomarker. Mult Scler. 2017 Jun;23(7):937-945], in combination with other clinical or paraclinical parameters.

Particularly, the main objective of the present invention is to improve the sensitivity and specificity associated with the use of sIFNAR2 as single biomarker for the diagnosis of MS. Consequently, in the present invention, logistic and discriminant regression of demographic, clinical and paraclinical variables were used, such as MRI, oligoclonal IgG bands (BOC IgG) in cerebrospinal fluid (CSF) and evoked potentials (EP), with the aim to improve the diagnostic sensitivity and specificity of sIFNAR2 as a biomarker found in the serum of treatment-naive MS patients versus healthy controls.

Thus, multiparametric interactions between sIFNAR2 and each of the following variables were analysed:

- Patient demographics (age, gender and other data).
- Baseline disease data (flare-ups in the previous year, baseline disability -time of sample collection- EDSS score, years of MS progression).
- Cerebrospinal fluid (CSF) data (BOC IgG + or -).
- MRI data, either baseline or previous, such as an MRI scan performed within the previous 12 months (spatial dissemination (SD) criteria/temporal dissemination (TD) criteria assessed by a multiple sclerosis expert).
- Data for visual evoked potentials (VEP) (latency: value) of right eye (RE) and left eye (LE).

To this end, a number of patients (43) and controls (43) were selected by random sampling ensuring that biomarker levels match the data from the reference study [Órpez-Zafra T, Pavía J, Hurtado-Guerrero I, Pinto Medel MJ, Rodriguez Bada JL, Urbaneja P, Suardíaz M, Villar LM, Comabella M, Montalban X, Alvarez-Cermeño JC, Leyva L, Fernandez Ó, Oliver-Martos B. Decreased soluble IFN-β receptor (sIFNAR2) in multiple sclerosis patients: A potential serum diagnostic biomarker. Mult Scler. 2017 Jun;23(7):937-945].

Subsequently, demographic, clinical and paraclinical (MRI, CSF, VEP) data were obtained (**Table 1**). **Table 1** shows that MS patients are representative of the disease (age, gender) and showed clinical activity within the previous year as flare-ups (1.0±0.9) with an EDSS score at the time of measurement indicating an early phase of the disease (mean EDSS±SD:1.6±1.4), although the EDSS range of 0.0 - 6.5 covers a broad spectrum of disability in patients with MS.

The results of the logistic regression analysis for variables included in the study are listed in **Table 2.** It must be noted that clinical and sIFNAR2 data did not show significant differences for SE/ES/PPV/NPV versus the reference study [Órpez-Zafra T, Pavía J, Hurtado-Guerrero I, Pinto-Medel MJ, Rodriguez Bada JL, Urbaneja P, Suardíaz M, Villar LM, Comabella M, Montalban X, Alvarez-Cermeño JC, Leyva L, Fernández Ó, Oliver-Martos B. Decreased soluble IFN-β receptor (sIFNAR2) in multiple sclerosis patients: A potential serum diagnostic biomarker. Mult Scler. 2017 Jun;23(7):937-945] confirming that the correct subsample was selected by random resampling.

Such as it is shown in **Table 2,** the combination of IFNAR2 with any of the following variables MRI_DS, MRI_DT or [MRI_DS+MRI_DT] always gave rise to an improved AUC value (between 0.91 and 0.95) as compared with both the AUC associated with the use of IFNAR2 alone (0.82) and the AUC associated with MRI_DS (0.79) or [MRI_DS+MRI_DT] (0.80).

So, an unexpected synergistic effect was observed when IFNAR2 was combined at least with MRI_DS and/or MRI_DT. These results are of utmost clinical relevance since, although MRI is the gold standard test for the diagnosis of MS in clinical practice, it has a sensitivity of around 80% and is, in turn, the cause of much of the diagnostic errors associated with the disease. However, the combination of MRI with sIFNAR2 levels offers a synergistic effect able to improve the individual AUC values of both MRI or sIFNAR2, and can be the basis of a specific test for the diagnosis of MS.

Moreover, when VEP values were combined with IFNAR2 and [MRI_DS and/or MRI_DT], high AUC values were also obtained. See for example the combinations of IFNAR2 with [MRI_DS+VEP], [MRI_DT+VEP] or [MRI_DS+MRI_DT+VEP] in **Table 2,** showing AUC values between 0.93 and 0.95.

Finally, when the assessment of the presence of BOC_IgG was included in the combinations, even higher AUC values were obtained. See for instance the combinations of IFNAR2 with [MRI_SD+VEP+BOC_IgG] or [MRI_SD+ MRI_TD+VEP+BOC_IgG] in **Table** 2 showing an AUC = 0.99).

So, the first embodiment of the present invention refers to an *in vitro* method (hereinafter *"method of the invention"*) for the diagnosis and/or prognosis of MS in a subject, which comprises assessing the concentration level of sIFNAR2 in a biological sample obtained from the subject in combination with the obtention of a MRI to assess the presence/absence of MRI_DS and/or MRI_DT (or in combination with data obtained from performing the MRI), wherein the identification of a lower level of sIFNAR2 as compared with a pre-established threshold level, and the presence of MRI_DS and/or MRI_DT, are indications that the subject is suffering from MS.

In a preferred embodiment, a MRI is performed to assess the presence of MRI_SD which means that inflammatory lesions have occurred in more than one already predefined locations in the central nervous system and/or to assess the presence of MRI_TD which means that new lesions has developed in the course of the disease. See the article [Alan J Thompson et al., 2017. Diagnosis of multiple sclerosis: 2017 revisions of the McDonald criteria. Lancet Neurol. 2018 Feb;17(2):162-173. doi: 10.1016/S1474-4422(17)30470-2. Epub 2017 Dec 21*]* showing McDonald criteria for demonstration of dissemination in space and time by MRI in a patient with a clinically isolated syndrome. Dissemination in space can be demonstrated by one or more T2-hyperintense lesions that are characteristic of multiple sclerosis in two or more of four areas of the CNS: periventricular, cortical or juxtacortical, and infratentorial brain regions, and the spinal cord. Dissemination in time can be demonstrated by the simultaneous presence of gadolinium-enhancing and non-enhancing lesions at any time or by a new T2-hyperintense or gadolinium-enhancing lesion on follow-up MRI, with reference to a baseline scan, irrespective of the timing of the baseline MRI.

In a preferred embodiment of the present invention, the method of the invention comprises assessing the concentration level of sIFNAR2 in a biological sample obtained from a patient who shows clinical and/or radiologic features suggestive of MS and is characterized by the presence of MRI_DS and/or MRI_DT identified by MRI.

In a preferred embodiment, the method of the invention further comprises the assessment of VEP values.

In a preferred embodiment, VEP values are determined by analysing either the latency, morphology and/or amplitude of any of the deflections P-100, N-75 y N-145. In a preferred embodiment VEP values are determined preferably by analysing the latency of the deflection P-100, wherein a delayed VEP latency, as compared with a pre-established VEP latency value, is an indication that the patient is suffering from MS. In a still preferred embodiment, the pre-established VEP latency value is obtained from healthy subjects. Typically, a VEP latency higher than 111.1 millisecond is an indication that the patient may be suffering from MS.

In a preferred embodiment, the method of the invention further comprises assessing the presence or absence of BOC_IgG, where the presence of at least two BOC_IgG in CSF, not present in serum, is an indication that the patient may be suffering from MS.

In a preferred embodiment, the method of the invention further comprises processing the concentration level of sIFNAR2, the information obtained from MRI images regarding the presence of MRI_DS and/or MRI_DT, VEP values, and information regarding the presence of at least two BOC_IgG in CSF, not present in serum , in order to define a risk score, wherein if a deviation or variation of the risk score value is identified, as compared with a reference value, this is indicative that the patient is suffering from MS.

In a preferred embodiment of the present invention, the determination of sIFNAR2 concentration level is carried out in a biological sample, preferably a biological fluid selected from blood, plasma, serum, cerebrospinal fluid, urine or tears.

In a preferred embodiment of the present invention, the determination of sIFNAR2 concentration level is carried out by means of an immunoassay, preferably by a technique selected from: immunoblot, enzyme-linked immunosorbent assay (ELISA), linear immunoassay (LIA), radioimmunoassay (RIA), immunofluorescence, x-map, protein chips or aptamer-based ELISA, or any technique for the determination of sIFNAR2 concentration level in a biological sample.

In a preferred embodiment of the present invention further comprises: a) Receiving by a computer program the concentration level of sIFNAR2, information regarding the presence of MRI_DS and/or MRI_DT, VEP values and information regarding the presence of at least two BOC_IgG in CSF, not present in serum; b) processing the information received according to the step a) for finding substantial variations or deviations with respect to the information obtained from a healthy subject, and c) providing an output through a terminal display when a variation or deviation is found which indicates that the subject might be suffering from MS.

The second embodiment of the present invention refers to the *in vitro* use of sIFNAR2, in combination with MRI measured variables MRI_DS and/or MRI_DT, for the diagnosis and/or prognosis of MS.

In a preferred embodiment of the present invention refer to the *in vitro* use of sIFNAR2 in combination with MRI measured variables MRI_DS and/or MRI_DT; and in combination with VEP values.

In a preferred embodiment of the present invention refer to the *in vitro* use of sIFNAR2 in combination with MRI measured variables MRI_DS and/or MRI_DT; and in combination with VEP values and at least two BOC_IgG in CSF, not present in serum.

The last embodiment of the present invention refers to a method for treating a patient suffering from MS (wherein the treatment is typically focused on speeding recovery from attacks, slowing the progression of the disease and managing MS symptoms) or, alternatively, assessing the treatment to be applied to a patient suffering from MS, wherein the patient has been diagnosed using the method of the invention.

For the purpose of the present invention, the following terms are defined:

- The term "comprising" means including, but not limited to, whatever follows the word "comprising". Thus, the use of the term "comprising" indicates that the listed elements are required or mandatory but that other elements are optional and may or may not be present.
- The term "consisting of" means including, and limited to, whatever follows the phrase "consisting of". Thus, the phrase "consisting of" indicates that the listed elements are required or mandatory and that no other elements may be present.
- The term "pre-established threshold level" typically refers to the level measured in healthy patients. The patient is likely to suffer from MS with a given sensitivity and specificity if the levels of the biomarker in the patient are above said "pre-established threshold level". A "threshold value" or "cut-off value" can be determined experimentally, empirically, or theoretically. A "threshold value" can also be arbitrarily selected based upon the existing experimental and/or clinical conditions, as would be recognized by a person of ordinary skilled in the art. The "threshold value" has to be determined in order to obtain the optimal sensitivity and specificity according to the function of the test and the benefit/risk balance (clinical consequences of false positive and false negative). Preferably, the person skilled in the art may compare the level of the biomarker obtained according to the method of the invention with a defined "threshold value". In one embodiment of the present invention, the "threshold value" is derived from the level of biomarker determined in a control sample derived from one or more subjects who are substantially healthy. In one embodiment of the present invention, the "threshold value" may also be derived from the level of the biomarker determined in a control sample derived from one or more subjects who suffers from MS. Furthermore, retrospective measurement of the level of the biomarker in properly banked historical subject samples may be used in establishing these "threshold values". Typically, the optimal sensitivity and specificity (and so the "threshold value") can be determined using a Receiver Operating Characteristic (ROC) curve based on experimental data. For example, after determining the levels of the biomarker in a group of reference, one can use algorithmic analysis for the statistic treatment of the measured concentrations of biomarkers in biological samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-speciftcity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is moderate. When AUC is higher than 0.9, the accuracy is quite high. This algorithmic method is preferably done with a computer. Existing software or systems in the art may be used for the drawing of the ROC curve.

### Description of the figures

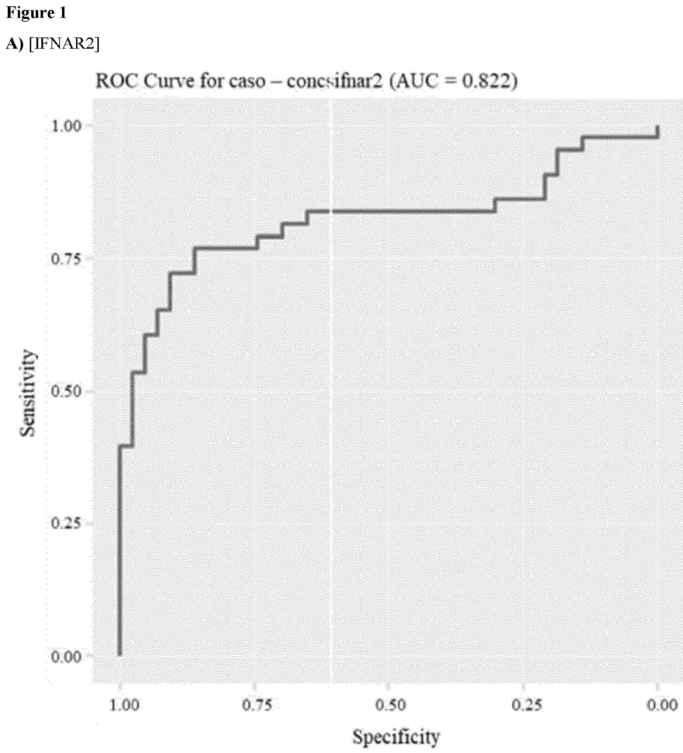

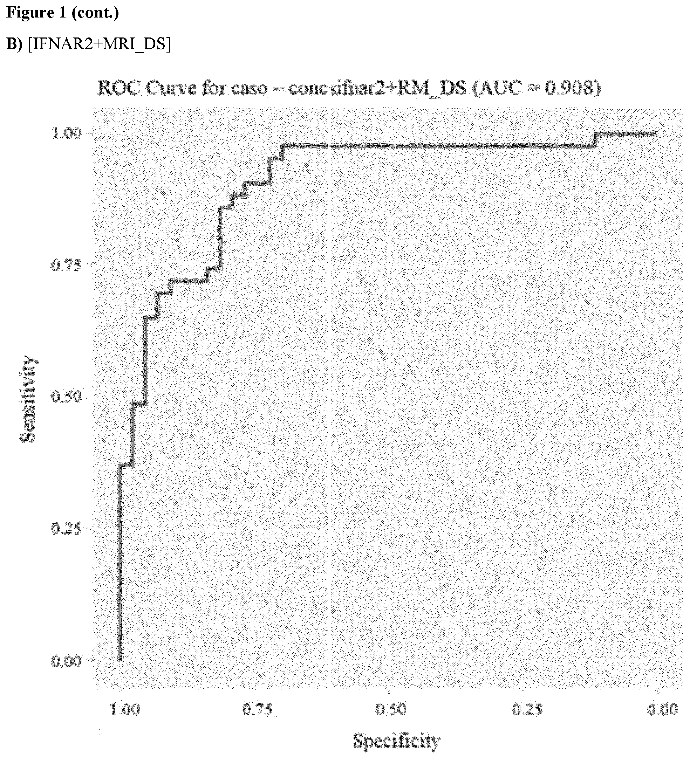

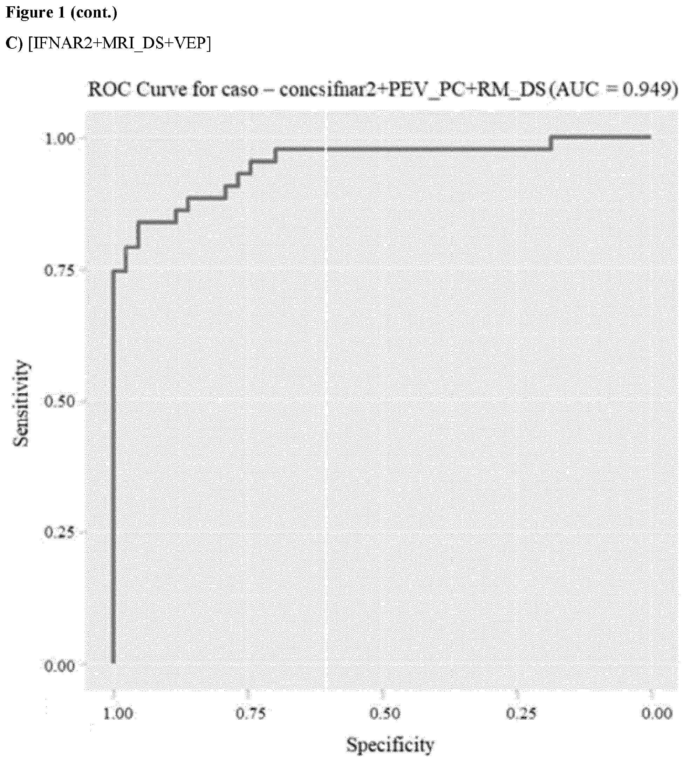

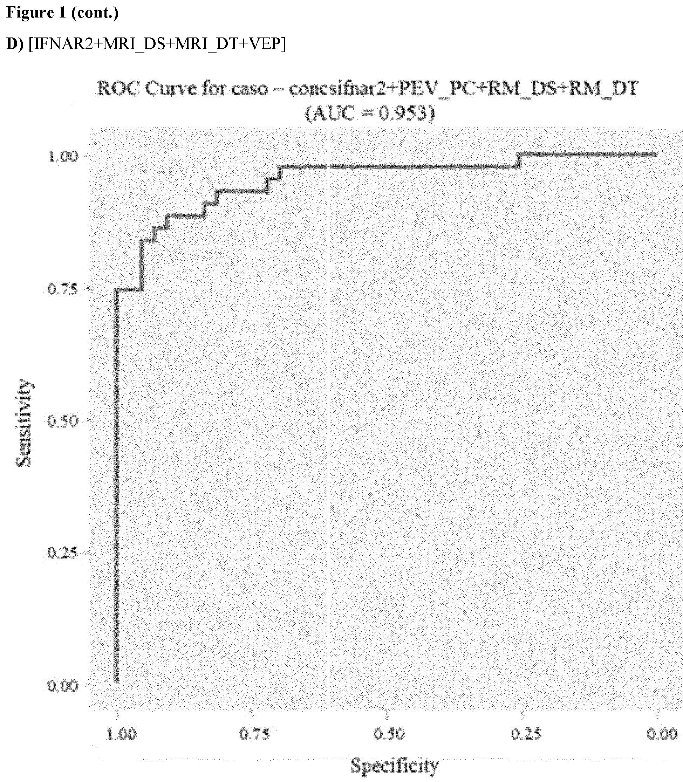

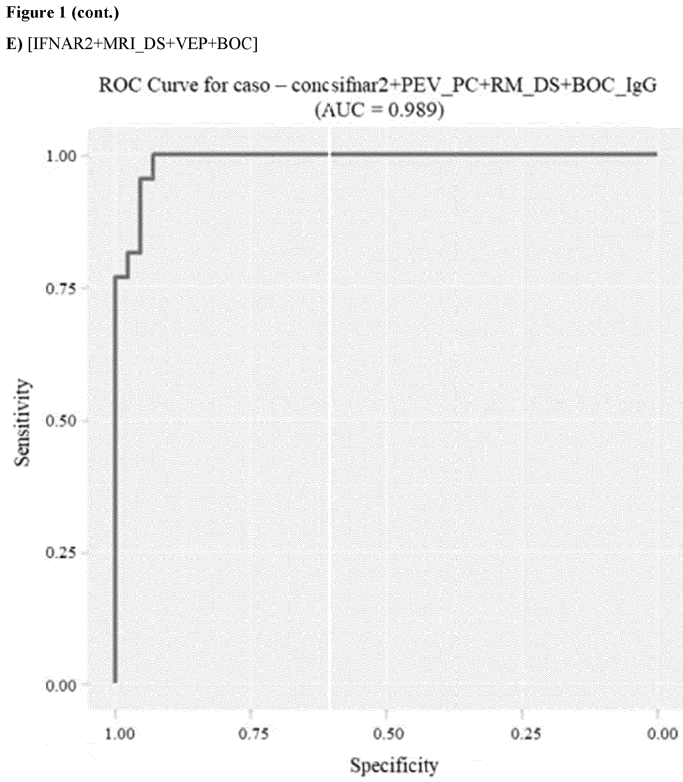

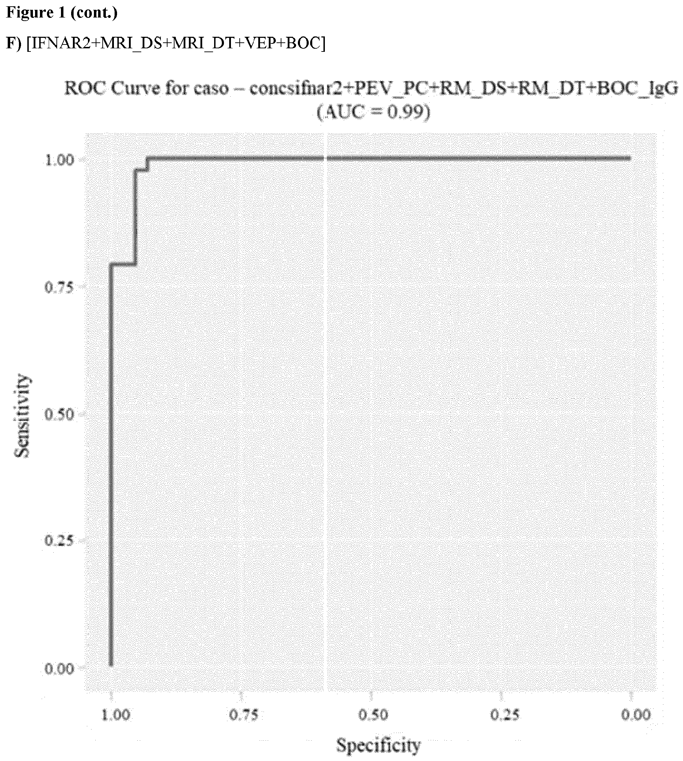

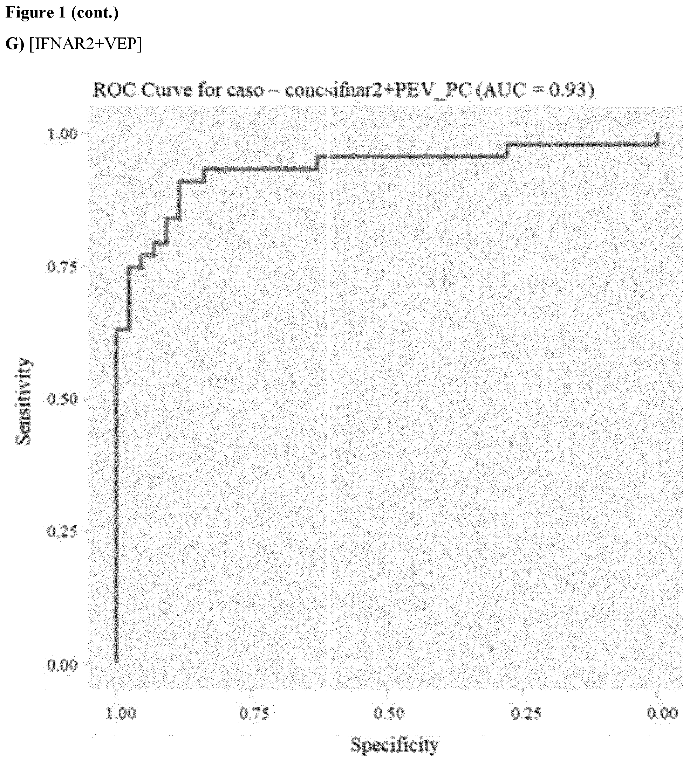

**Figure** 1. It shows ROC curves representing the AUC value for each of the following signatures. X axis (Specificity). Y axis (Sensitivity).

A)

$$[\text{IFNAR2}] > \text{AUC} = 0.82.$$

B)

$$[\text{IFNAR2+MRI\_DS}] > \text{AUC} = 0.91.$$

C)

$$[IFNAR2+MRI\_DS+VEP] > AUC = 0.95.$$

D)

$$[IFNAR2+MRI\_DS+MRI\_DT+VEP] > AUC = 0.95.$$

E)

$$[IFNAR2+MRI\_DS+VEP+BOC] > AUC = 0.99.$$

F)

$$[IFNAR2+MRI\_DS+MRI\_DT+VEP+BOC > AUC = 0.99.$$

G)

$$[IFNAR2+VEP] > AUC = 0.93.$$

**Detailed description of the invention**

The present invention is illustrated by means of the Examples set below without the intention of limiting its scope of protection.

**Example 1. Material and methods**

**Example 1.1. Sample selection**

These have been selected by random resampling of a data subsample from 43 non-treated patients and 43 healthy controls who participated in a previous referenced study, seeking data samples that have similar mean sIFNAR2 values as those reported in the reference publication [Órpez-Zafra T, Pavia J, Hurtado-Guerrero I, Pinto-Medel MJ, Rodriguez Bada JL, Urbaneja P, Suardíaz M, Villar LM, Comabella M, Montalban X, Alvarez-Cermeño JC, Leyva L, Fernández Ó, Oliver-Martos B. Decreased soluble IFN-β receptor (sIFNAR2) in multiple sclerosis patients: A potential serum diagnostic biomarker. Mult Scler. 2017 Jun; 23(7):937-945].

A database including demographic and clinical data was constructed. Data was obtained from medical records and analysis of paraclinical examinations (MRI, VEP and CSF) of the 43 patients and 43 controls. The sIFNAR2 data of selected patients and controls were obtained from the reference study [Órpez-Zafra T, Pavía J, Hurtado-Guerrero I, Pinto-Medel MJ, Rodriguez Bada JL, Urbaneja P, Suardíaz M, Villar LM, Comabella M, Montalban X, Alvarez-Cermeño JC, Leyva L, Fernández Ó, Oliver-Martos B. Decreased soluble IFN-β receptor (sIFNAR2) in multiple sclerosis patients: A potential serum diagnostic biomarker. Mult Scler. 2017 Jun;23(7):937-945].

**Example 1.2. Statistics**

A multivariate analysis by logistic and discriminant regression of the demographic, clinical and paraclinical variables (MRI, CSF, VEP) included in the study was performed to obtain the best predictors from sIFNAR2 and the rest of markers (sensitivity (SE), specificity (SP), positive predictive value (PPV) and negative predictive value (NPV)), each of them alone and in various combinations. The area under the curve (AUC) - ROC curve was used to select the best classifier models, in order to improve the diagnostic SE and SP of sIFNAR2 as a biomarker in the serum of treatment-naive MS patients versus healthy controls. Results are shown in **Table 2.**

For MRI sensitivity and specificity data were obtained from a reference publication [Barkhof F, Filippi M, Miller DH et al. Comparison of MRI criteria at first presentation to predict conversion to clinically definite multiple sclerosis. Brain 1997;120(Pt11):2059-2069] and for BOC-IgG in CSF [Abraira V, Alvarez-Cermeño JC, Arroyo R, Cámara C, Casanova B, Cubillo S, et al. Utility of oligoclonal IgG band detection for MS diagnosis in daily clinical practice. J Immunol Methods. 2011 Aug 31;371(1-2):170-173], . For VEP values in controls, data was obtained from 43 healthy subjects used as normality reference values by the Clinical Neurophysiology Laboratory at HRUM and from a reference publication [Thabit1 MN, Farouk MM, Awni M, Mohamed AB. Early disability in ambulatory patients with multiple sclerosis: optical coherence tomography versus visual evoked potentials, a comparative study. Egyptian J Neurol, Psychiat and Neurosurg (2020) 56:70]. For all variables, all values obtained at HRUM were maintained, including extreme values and avoiding any manipulation of the data.

**Example 1.3. Ethical considerations treatment of personal data**

This study was conducted with the prior approval of the study site's IRB (Malaga Regional University Hospital - HRUM), following the guidelines of the Declaration of Helsinki, 1964, Belmont Report, 1978, EU Regulation 2016/679 on Data Protection (GDPR), the Organic Law 3/2018, of 5 December, on the Protection of Personal Data and Guarantee of Digital Rights (LOPDGDD) and Royal Decree 1716/2011, of 18 November, which establishes the basic requirements for the authorization and operation of biobanks for biomedical research purposes and the treatment of biological samples of human origin, and regulating the operation of the National Register of Biobanks for biomedical research, the Law 41/2002, of 14 November 2002, which regulates patient autonomy and the rights and obligations regarding information and clinical documentation, the Law 14/2007, of 3 July, on Biomedical Research, the Standards of Good Clinical Practice (GCP) of the International Conference on Harmonization (ICH), as well as any other applicable standard and/or law.

**Example 2. Results**

**Example 1. Demographical, clinical and paraclinical results**

Demographical, clinical and paraclinical results are shown in **Table 1** (see below). Data included in **Table 1** show a typical population of MS patients in terms of demographics: age 37.8 (13.9), female 60.5%. Baseline EDSS 1.6 (1.4), range 0.0-6.5.

**Table 1**

| Variable | PATIENTS (N=43) | CONTROLS (N=43) | Total (N=86) | p value |
|---|---|---|---|---|
| | | | | |
| **Age** | | | | |
| **N-Miss** | 0.0 | 43.0 | 43.0 | |
| **Mean (SD)** | 37.8 (13.9) | N/A | 37.8 (13.9) | |
| **Range** | 14.0 - 66.0 | N/A | 14.0 - 66.0 | |
| **gender** | | | | |
| **N-Miss** | 0.0 | 43.0 | 43.0 | |
| **Male** | 17.0 (39.5%) | 0.0 | 17.0 (39.5%) | |
| **Female** | 26.0 (60.5%) | 0.0 | 26.0 (60.5%) | |
| **Flare-ups** | | | | |
| **N-Miss** | 2.0 | 43.0 | 45.0 | |
| **Mean (SD)** | 1.0 (0.9) | N/A | 1.0 (0.9) | |
| **Range** | 0.0-3.0 | N/A | 0.0-3.0 | |
| **Baseline EDSS** | | | | |
| **N-Miss** | 7.0 | 43.0 | 50.0 | |
| **Mean (SD)** | 1.6 (1.4) | N/A | 1.6 (1.4) | |
| **Range VEP_HIGHEST** | 0.0-6.5 | N/A | 0.0 - 6.5 | <0.0011 |
| **Mean (SD)** | 114.9 (11.0) | 100.8 (6.6) | 107.8 (11.5) | |
| **Range MVEP** | 91.0 - 150.0 | 88.7 - 112.1 | 88.7 - 150.0 | <0.0011 |
| **N-Miss** | 5.0 | 0.0 | 5.0 | |
| **Mean (SD)** | 111.0 (10.0) | 98.0 (6.5) | 104.1 (10.5) | |

(continued)

| Variable | PATIENTS (N=43) | CONTROLS (N=43) | Total (N=86) | p value |
|---|---|---|---|---|
| Range VEP_PC111.1 | 81.5 -135.0 | 86.7 -110.0 | 81.5 -135.0 | <0.0012 |
| N-Miss | 5.0 | 0.0 | 5.0 | |
| 1 | 16.0 (42.1%) | 42.0 (97.7%) | 58.0 (71.6%) | |
| 2 Abnormal MRI_SD | 22.0 (57.9%) | 1.0 (2.3%) | 23.0 (28.4%) | <0.0012 |
| 0.0 | 6.0 (14.0%) | 34.0 (79.1%) | 40.0 (46.5%) | |
| 0.8 | 13.0 (30.2%) | 0.0 (0.0%) | 13.0 (15.1%) | |
| 1.0 Yes MRI_TD | 24.0 (55.8%) | 9.0 (20.9%) | 33.0 (38.4%) | <0.0012 |
| 0.000 | 13.0 (30.2%) | 26.0 (60.5%) | 39.0 (45.3%) | |
| 0.581 | 12.0 (27.9%) | 0.0 (0.0%) | 12.0 (14.0%) | |
| 1,000 Yes BOC_IgG | 18.0 (41.9%) | 17.0 (39.5%) | 35.0 (40.7%) | <0.0012 |
| 1.000 Positive | 37.0 (86.0%) | 3.0 (7.0%) | 40.0 (46.5%) | |
| 1.075 | 3.0 (7.0%) | 0.0 (0.0%) | 3.0 (3.5%) | |
| 2.000 | 3.0 (7.0%) | 40.0 (93.0%) | 43.0 (50.0%) | |

*1. Linear Model ANOVA. 2. Pearson's Chi-squared test*

For VEP values in controls, data from 43 healthy subjects used as reference by the Clinical Neurophysiology Laboratory at HRUM and a reference publication were used to set the normality threshold [Thabit1 MN, Farouk MM, Awni M, Mohamed AB. Early disability in ambulatory patients with multiple sclerosis: optical coherence tomography versus visual evoked potentials, a comparative study. Egyptian J Neurol, Psychiat and Neurosurg (2020) 56:70], which was set as the mean $\pm$ 2SD, as specified in the literature. The highest VEP latency (including RE and LE) for patients was 114.9 (11.0), and for controls was 100.8 (6.6). The mean VEP latency for patients was 111.0 (10.0), and for controls was 98.0 (6.5). Patients showed an abnormal VEP_PC111_1 threshold (latency $111 \pm 2SD$) in 57.9% of cases, while controls showed an abnormal threshold only in 2.3% of cases.

For MRI and CSF data of controls, SE and SP data from two published reference studies were used [Barkhof F, Filippi M, Miller DH et al. Comparison of MRI criteria at first presentation to predict conversion to clinically definite multiple sclerosis. Brain 1997;120(Pt11):2059-2069] and [Abraira V, Alvarez-Cermeño JC, Arroyo R, Cámara C, Casanova B, Cubillo S, et al. Utility of oligoclonal IgG band detection for MS diagnosis in daily clinical practice. J Immunol Methods. 2011 Aug 31;371(1-2):170-173].

All values obtained at HRUM for all variables have been maintained, including extreme values, avoiding any manipulation of the data.

## Example 2. Results of the logistic regression analysis of paraclinical variables

The results of the logistic regression analysis of paraclinical variables are shown in **Table 2.**

Table 2

| Variable | AUC | Sensitivity | Specificity | PPV | NPV |
|---|---|---|---|---|---|
| IFNAR2 | 0.82 | 76.74 | 76.74 | 76.74 | 76.74 |
| MRI_DS | 0.79 | 86.05 | 79.07 | 80.43 | 85 |
| MRI_DS+MRI_DT | 0.80 | 86.05 | 79.07 | 80.43 | 85 |
| IFNAR2+MRI_DS | 0.91 | 90.70 | 76.74 | 79.59 | 89.19 |
| IFNAR2+MRI_DT | 0.91 | 93.02 | 79.07 | 81.63 | 91.89 |

(continued)

| Variable | AUC | Sensitivity | Specificity | PPV | NPV |
|---|---|---|---|---|---|
| IFNAR2+MRI_DS+MRI_DT | 0.95 | 86.05 | 93.02 | 92.50 | 86.96 |
| IFNAR2+MRI_DS+VEP | 0.95 | 83.72 | 95.35 | 94.74 | 85.42 |
| IFNAR2+MRI_DT+VEP | 0.93 | 90.70 | 88.37 | 88.64 | 90.48 |
| IFNAR2+MRI_DS+MRI_DT+VEP | 0.95 | 86.05 | 93.02 | 92.50 | 86.96 |
| IFNAR2+MRI_DS+MRI_DT+VEP+BOC_IgG | 0.99 | 97.67 | 93.02 | 93.33 | 97.56 |
| IFNAR2+MRI_DS+VEP+BOC_IgG | 0.99 | 100 | 93.02 | 93.48 | 100 |
| IFNAR2+VEP | 0.93 | 90.69 | 88.37 | 88.64 | 90.58 |

Clinical and sIFNAR2 data did not show significant differences for SE/SP/PPV/NPV versus the reference study, [Órpez-Zafra T, Pavia J, Hurtado-Guerrero I, Pinto-Medel MJ, Rodriguez Bada JL, Urbaneja P, Suardíaz M, Villar LM, Comabella M, Montalban X, Alvarez-Cermeño JC, Leyva L, Fernández Ó, Oliver-Martos B. Decreased soluble IFN-β receptor (sIFNAR2) in multiple sclerosis patients: A potential serum diagnostic biomarker. Mult Scler. 2017 Jun;23(7):937-945], confirming that the correct subsample was selected by random resampling. All SE/ES/PPV/NPV values ranged around 77%.

For VEP values, the cut-off point was set as the mean±2SD of healthy controls (latency of VEP_PC111.1=111.1 milliseconds), as described in the literature. This value is consistent with data reported in the literature [Thabit1 MN, Farouk MM, Awni M, Mohamed AB. Early disability in ambulatory patients with multiple sclerosis: optical coherence tomography versus visual evoked potentials, a comparative study. Egyptian J Neurol, Psychiat and Neurosurg (2020) 56:70].

When serum sIFNAR2 and VEP_PC111.1 were jointly evaluated with this cut-off point, SE/ES/PPV/NPV values increased to around 90%.

SE/ES/PPV/NPV values increased with the number of variables included in the predictive model, and as might be expected, inclusion of BOC-IgG values and MRI spatial dissemination criteria (MRI_SD) was particularly useful.

Such as it is shown in **Table 2,** the combination of IFNAR2 with any of the following variables MRI_DS, MRI_DT or [MRI_DS+MRI_DT] always gave rise to an improved AUC value (between 0.90 and 0.95) as compared with both the AUC associated with the use of IFNAR2 alone (0.82) and the AUC associated with MRI_DS (0.79) or [MRI_DS+MRI_DT] (0.80).

So, an unexpected synergistic effect was observed when IFNAR2 was combined at least with MRI_DS and/or MRI_DT. These results are of utmost clinical relevance since, although MRI is the gold standard test for the diagnosis of MS in clinical practice, it has a sensitivity of around 80% and is, in turn, the cause of much of the diagnostic errors associated with the disease. However, the combination of MRI with sIFNAR2 levels offers a synergistic effect able to improve the individual AUC values of both MRI or sIFNAR2, and can be the basis of a specific test for the diagnosis of MS.

Moreover, when VEP values were combined with IFNAR2 and [MRI_DS and/or MRI_DT], high AUC values were also obtained. See for example the combinations of IFNAR2 with [MRI_DS+VEP], [MRI_DT+VEP] or [MRI_DS+MRI_DT+VEP] in **Table 2,** showing AUC values between 0.93 and 0.95.

Finally, when the assessment of the presence of BOC_IgG in CSF was included in the combinations, even higher AUC values were obtained. See for instance the combinations of IFNAR2 with [MRI_SD+VEP+BOC_IgG] or [MRI_SD+MRI_TD+VEP+BOC_IgG] in **Table** 2 showing an AUC = 0.99).

## Claims

1. *In vitro* method for the diagnosis and/or prognosis of multiple sclerosis (MS) in a subject, which comprises assessing the concentration level of sIFNAR2 in a biological sample obtained from the subject in combination with the obtention of a magnetic resonance imaging (MRI) to assess the presence/absence of dissemination in space (MRI_DS) and/or dissemination in time (MRI_DT), wherein the identification of a lower level of sIFNAR2 as compared with a pre-established threshold level, and the presence of MRI_DS and/or MRI_DT, are indications that the subject is suffering from MS.

2. *In vitro* method, according to claim 1, which comprises assessing the concentration level of sIFNAR2 in a biological sample obtained from a patient, wherein the patient shows clinical and/or radiologic features suggestive of MS and is **characterized by** the presence of MRI_DS and/or MRI_DT identified by MRI.

3. *In vitro* method, according to any of the previous claims, further comprising the assessment of visual evoked potential (VEP) values.

4. *In vitro* method, according to any of the previous claims, further comprising the assessment of the presence or absence of oligoclonal IgG bands (BOC_IgG) in CSF.

5. *In vitro* method, according to any of the previous claims, which further comprises processing the concentration level of sIFNAR2, the information obtained from MRI images regarding the presence of MRI_DS and/or MRI_DT, VEP values and information regarding the presence of BOC_IgG in CSF in order to define a risk score, wherein if a deviation or variation of the risk score value is identified, as compared with a reference value, this is indicative that the patient is suffering from MS.

6. *In vitro* method, according to any of the previous claims, wherein the biological sample is a biological fluid selected from blood, plasma, serum, cerebrospinal fluid, urine or tears.

7. *In vitro* method, according to any of the previous claims, wherein the determination of sIFNAR2 concentration level is carried out by means of an immunoassay, preferably by a technique selected from: immunoblot, enzyme-linked immunosorbent assay (ELISA), linear immunoassay (LIA), radioimmunoassay (RIA), immunofluorescence, x-map, protein chips or aptamer-based ELISA, or any technique for the determination of sIFNAR2 concentration level in a biological sample.

8. *In vitro* method, according to any of the previous claims, further comprising:

   a. Receiving by a computer program the concentration level of sIFNAR2, information regarding the presence of MRI_DS and/or MRI_DT, VEP values and information regarding the presence of BOC_IgG in CSF.
   b. Processing the information received according to the step a) for finding substantial variations or deviations with respect to the information obtained from a healthy subject, and
   c. Providing an output through a terminal display when a variation or deviation is found which indicates that the subject might be suffering from MS.

9. *In vitro* use of sIFNAR2 in a biological sample obtained from the subject, in combination with MRI measured variables MRI_DS and/or MRI_DT, for the diagnosis and/or prognosis of MS.

10. *In vitro* use, according to claim 9, of sIFNAR2 in combination with MRI measured variables MRI_DS and/or MRI_DT; and in combination with VEP values.

11. *In vitro* use, according to any of the claims 9 or 10, of sIFNAR2 in combination with MRI measured variables MRI_DS and/or MRI_DT, VEP values and BOC_IgG in CSF.

**Patentansprüche**

1. *In-vitro*-Verfahren zur Diagnose und/oder Prognose von multipler Sklerose (MS) in einem Individuum, welches das Beurteilen des Konzentrationsniveaus von sIFNAR2 in einer biologischen Probe erhalten aus dem Individuum in Kombination mit dem Erhalten einer Magnetresonanzbildgebung (MRI) umfasst, um die Anwesenheit/Abwesenheit von räumlicher Dissemination (MRI_DS) und/oder zeitlicher Dissemination (MRI_DT) zu beurteilen, wobei die Identifizierung eines niedrigeren Niveaus von sIFNAR2 im Vergleich zu einem im Voraus festgesetzten Schwellenniveau, und die Anwesenheit von MRI_DS und/oder MRI_DT, Anzeichen dafür sind, dass das Individuum unter MS leidet.

2. *In-vitro*-Verfahren nach Anspruch 1, welches das Beurteilen des Konzentrationsniveaus von sIFNAR2 in einer biologischen Probe erhalten aus einem Patienten umfasst, wobei der Patient klinische und/oder radiologische Merkmale, welche auf MS hindeuten, zeigt und durch die Anwesenheit von MRI_DS und/oder MRI_DT, identifiziert mittels MRI, gekennzeichnet ist.

3. *In-vitro*-Verfahren nach einem der vorhergehenden Ansprüche, zusätzlich umfassend die Beurteilung von Werten des visuell evozierten Potenzials (VEP).

4. *In-vitro*-Verfahren nach einem der vorhergehenden Ansprüche, zusätzlich umfassend die Beurteilung der Anwesenheit oder Abwesenheit von oligoklonalen IgG-Banden (BOC_IgG) in CSF.

5. *In-vitro*-Verfahren nach einem der vorhergehenden Ansprüche, welches zusätzlich das Bearbeiten des Konzentrationsniveaus von sIFNAR2, der Information erhalten aus MRI-Bildern bezüglich der Anwesenheit von MRI_DS und/oder MRI_DT, der VEP-Werte und der Information bezüglich der Anwesenheit von BOC_IgG in CSF umfasst, um eine Risikopunktzahl zu definieren, wobei, wenn eine Abweichung oder Variation des Wertes der Risikopunktzahl, im Vergleich zu einem Referenzwert, identifiziert wird, dies darauf schließen lässt, dass der Patient unter MS leidet.

6. *In-vitro*-Verfahren nach einem der vorhergehenden Ansprüche, wobei die biologische Probe ein biologisches Fluid ausgewählt aus Blut, Plasma, Serum, Gehirn-Rückenmark-Flüssigkeit, Urin oder Tränen ist.

7. *In-vitro*-Verfahren nach einem der vorhergehenden Ansprüche, wobei die Bestimmung des Konzentrationsniveaus von sIFNAR2 mittels eines Immunassays vorgenommen wird, vorzugsweise mittels einer Technik ausgewählt aus: Immunblot, enzymverbundenem Immun-Sorptions-Versuch (ELISA), Line-Immunassay (LIA), Radioimmuntest (RIA), Immunfluoreszenz, x-Map, Proteinchips oder aptamerbasiertem ELISA, oder einer Technik für die Bestimmung des Konzentrationsniveaus von sIFNAR2 in einer biologischen Probe.

8. *In-vitro*-Verfahren nach einem der vorhergehenden Ansprüche, zusätzlich umfassend:

a. das Empfangen mittels eines Computerprogramms des Konzentrationsniveaus von sIFNAR2, der Information bezüglich der Anwesenheit von MRI_DS und/oder MRI_DT, der VEP-Werte und der Information bezüglich der Anwesenheit von BOC_IgG in CSF,
b. das Bearbeiten der gemäß Schritt a) empfangenen Information zum Herausfinden von wesentlichen Variationen oder Abweichungen in Bezug auf die Information erhalten aus einem gesunden Individuum, und
c. das Bereitstellen einer Ausgabe über eine Terminalanzeige, wenn eine Variation oder Abweichung gefunden wird, welche darauf schließen lässt, dass das Individuum unter MS leiden könnte.

9. *In-vitro*-Verwendung von sIFNAR2 in einer biologischen Probe erhalten aus dem Individuum, in Kombination mit den mittels MRI gemessenen Variablen MRI_DS und/oder MRI_DT, zur Diagnose und/oder Prognose von MS.

10. *In-vitro*-Verwendung nach Anspruch 9, von sIFNAR2 in Kombination mit den mittels MRI gemessenen Variablen MRI_DS und/oder MRI_DT; und in Kombination mit VEP-Werten.

11. *In-vitro*-Verwendung nach einem der Ansprüche 9 oder 10, von sIFNAR2 in Kombination mit den mittels MRI gemessenen Variablen MRI_DS und/oder MRI_DT, VEP-Werten und BOC_IgG in CSF.

## Revendications

1. Procédé *in vitro* de diagnostic et/ou de pronostic de la sclérose en plaques (SP) chez un sujet, qui comprend l'évaluation du niveau de concentration de sIFNAR2 dans un échantillon biologique obtenu du sujet en combinaison avec l'obtention d'une imagerie par résonance magnétique (IRM) pour évaluer la présence/absence de dissémination spatiale (IRM_DS) et/ou de dissémination temporelle (IRM_DT), dans lequel l'identification d'un niveau inférieur de sIFNAR2 par rapport à un niveau seuil préétabli, et la présence d'IRM_DS et/ou d'IRM_DT, sont des indications que le sujet souffre de SP.

2. Procédé *in vitro,* selon la revendication 1, qui comprend l'évaluation du niveau de concentration de sIFNAR2 dans un échantillon biologique obtenu d'un patient, dans lequel le patient présente des caractéristiques cliniques et/ou radiologiques suggérant la SP et est **caractérisé par** la présence d'IRM_DS et/ou d'IRM_DT identifiées par IRM.

3. Procédé *in vitro,* selon l'une quelconque des revendications précédentes, comprenant en outre l'évaluation de valeurs de potentiel évoqué visuel (PEV).

4. Procédé *in vitro,* selon l'une quelconque des revendications précédentes, comprenant en outre l'évaluation de la présence ou l'absence de bandes d'IgG oligoclonales (BOC_IgG) dans le LCR.

5. Procédé *in vitro,* selon l'une quelconque des revendications précédentes, qui comprend en outre le traitement du niveau de concentration de sIFNAR2, des informations obtenues à partir des images d'IRM concernant la présence de d'IRM_DS et/ou d'IRM_DT, des valeurs de PEV et des informations concernant la présence de BOC_IgG dans le LCR, afin de définir un score de risque, dans lequel, si une déviation ou variation de la valeur du score de risque est identifiée, par rapport à une valeur de référence, cela est indicatif que le patient souffre de SP.

6. Procédé *in vitro,* selon l'une quelconque des revendications précédentes, dans lequel l'échantillon biologique est un liquide biologique choisi parmi le sang, le plasma, le sérum, le liquide céphalo-rachidien, l'urine ou les larmes.

7. Procédé *in vitro,* selon l'une quelconque des revendications précédentes, dans lequel la détermination du niveau de concentration de sIFNAR2 est réalisée par le biais d'un immunoessai, de préférence par une technique choisie parmi : l'immunotransfert, l'essai d'immunoadsorption par enzyme liée (ELISA), l'immunoessai linéaire (LIA), le radioimmunoessai (RIA), l'immunofluorescence, le x-map, les chips de protéines ou l'ELISA à base d'aptamères, ou toute technique pour la détermination du niveau de concentration de sIFNAR2 dans un échantillon biologique.

8. Procédé *in vitro,* selon l'une quelconque des revendications précédentes, comprenant en outre :

   a. recevoir, par un programme informatique, le niveau de concentration de sIFNAR2, des informations concernant la présence d'IRM_DS et/ou d'IRM_DT, des valeurs de PEV et des informations concernant la présence de BOC_IgG dans le LCR ;
   b. traiter les informations reçues selon l'étape a) pour trouver des variations ou des déviations substantielles par rapport aux informations obtenues à partir d'un sujet sain, et
   c. fournir une sortie à travers un dispositif d'affichage de terminal lorsqu'une variation ou déviation est trouvée, ce qui indique que le sujet peut souffrir de SP.

9. Utilisation *in vitro* de sIFNAR2 dans un échantillon biologique obtenu du sujet, en combinaison avec des variables mesurées par IRM, l'IRM_DS et/ou l'IRM_DT, pour le diagnostic et/ou le pronostic de SP.

10. Utilisation *in vitro,* selon la revendication 9, de sIFNAR2 en combinaison avec des variables mesurées par IRM, l'IRM_DS et/ou l'IRM_DT ; et en combinaison avec des valeurs de PEV.

11. Utilisation *in vitro,* selon l'une quelconque des revendications 9 ou 10, de sIFNAR2 en combinaison avec des variables mesurées par IRM, l'IRM_DS et/ou l'IRM_DT, des valeurs de PEV et BOC_IgG dans le LCR.

**Figure 1**

**A)** [IFNAR2]

ROC Curve for caso – concsifnar2 (AUC = 0.822)

**Figure 1 (cont.)**

**B)** [IFNAR2+MRI_DS]

ROC Curve for caso – conc sifnar2+RM_DS (AUC = 0.908)

**Figure 1 (cont.)**

**C)** [IFNAR2+MRI_DS+VEP]

ROC Curve for caso – concsifnar2+PEV_PC+RM_DS (AUC = 0.949)

**Figure 1 (cont.)**

**D)** [IFNAR2+MRI_DS+MRI_DT+VEP]

ROC Curve for caso – conc sifnar2+PEV_PC+RM_DS+RM_DT (AUC = 0.953)

**Figure 1 (cont.)**

**E)** [IFNAR2+MRI_DS+VEP+BOC]

ROC Curve for caso – conc sifnar2+PEV_PC+RM_DS+BOC_IgG (AUC = 0.989)

**Figure 1 (cont.)**

**F)** [IFNAR2+MRI_DS+MRI_DT+VEP+BOC]

ROC Curve for caso – concsifnar2+PEV_PC+RM_DS+RM_DT+BOC_IgG (AUC = 0.99)

**Figure 1 (cont.)**

**G)** [IFNAR2+VEP]

ROC Curve for caso – concsifnar2+PEV_PC (AUC = 0.93)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016011212 A

**Non-patent literature cited in the description**

- **MARTINELLI V et al.** Multiple biomarkers improve the prediction of multiple sclerosis in clinically isolated syndromes. *ACTA NEUROLOGICA SCANDINAVICA*, 09 April 2017, vol. 136 (5), ISSN 0001-6314, 454-461
- **ÓRPEZ-ZAFRA T** ; **PAVÍA J** ; **PINTO-MEDEL MJ** ; **HURTADO-GUERRERO I** ; **RODRIGUEZ-BADA JL** ; **MARTIN MONTAÑEZ E** ; **FERNÁNDEZ Ó** ; **LEYVA L** ; **OLIVER-MARTOS B.** Development and validation of an ELISA for quantification of soluble IFN-β receptor: assessment in multiple sclerosis.. *Bioanalysis.*, 2015, vol. 7 (22), 2869-2880
- **ÓRPEZ-ZAFRA T** ; **PAVÍA J** ; **HURTADO-GUERRERO I** ; **PINTO-MEDEL MJ** ; **RODRIGUEZ BADA JL** ; **URBANEJA P** ; **SUARDÍAZ M** ; **VILLAR LM** ; **COMABELLA M** ; **MONTALBAN X**. Decreased soluble IFN-β receptor (sIFNAR2) in multiple sclerosis patients: A potential serum diagnostic biomarker.. *Mult Scler.*, June 2017, vol. 23 (7), 937-945
- **ÓRPEZ-ZAFRA T** ; **PAVÍA J** ; **HURTADO-GUERRERO I** ; **PINTO MEDEL MJ** ; **RODRIGUEZ BADA JL** ; **URBANEJA P** ; **SUARDÍAZ M** ; **VILLAR LM** ; **COMABELLA M** ; **MONTALBAN X**. Decreased soluble IFN-β receptor (sIFNAR2) in multiple sclerosis patients: A potential serum diagnostic biomarker.. *Mult Scler.*, June 2017, vol. 23 (7), 937-945
- **ALAN J THOMPSON et al.** Diagnosis of multiple sclerosis: 2017 revisions of the McDonald criteria.. *Lancet Neurol.*, 21 December 2017, vol. 17 (2), 162-173
- **ÓRPEZ-ZAFRA T** ; **PAVIA J** ; **HURTADO-GUERRERO I** ; **PINTO-MEDEL MJ** ; **RODRIGUEZ BADA JL** ; **URBANEJA P** ; **SUARDÍAZ M** ; **VILLAR LM** ; **COMABELLA M** ; **MONTALBAN X**. Decreased soluble IFN-β receptor (sIFNAR2) in multiple sclerosis patients: A potential serum diagnostic biomarker.. *Mult Scler.*, June 2017, vol. 23 (7), 937-945
- **BARKHOF F** ; **FILIPPI M** ; **MILLER DH et al.** Comparison of MRI criteria at first presentation to predict conversion to clinically definite multiple sclerosis.. *Brain*, 1997, vol. 120 (11), 2059-2069
- **ABRAIRA V** ; **ALVAREZ-CERMEÑO JC** ; **ARROYO R** ; **CÁMARA C** ; **CASANOVA B** ; **CUBILLO S et al.** Utility of oligoclonal IgG band detection for MS diagnosis in daily clinical practice.. *J Immunol Methods.*, 31 August 2011, vol. 371 (1-2), 170-173
- **THABIT1 MN** ; **FAROUK MM** ; **AWNI M** ; **MOHAMED AB.** Early disability in ambulatory patients with multiple sclerosis: optical coherence tomography versus visual evoked potentials, a comparative study.. *Egyptian J Neurol, Psychiat and Neurosurg*, 2020, vol. 56, 70